# EUROPEAN PATENT SPECIFICATION

(11) **EP 3 344 256 B1**
(45) Date of publication and mention of the grant of the patent: **05.08.2020**
(21) Application number: 16842883.7
(22) Date of filing: 31.08.2016
(51) Int. Cl.: A61K 31/549, A61K 31/728, A61P 19/02, A61K 9/00, A61K 9/06, A61K 9/51, A61K 47/36, A61L 27/20, A61L 27/52, A61L 27/54, A61K 38/18

(54) **COMPOSITIONS FOR THE TREATMENT OF JOINTS**
ZUSAMMENSETZUNGEN ZUR BEHANDLUNG VON GELENKEN
COMPOSITIONS DESTINÉES AU TRAITEMENT DES ARTICULATIONS

(30) Priority: 31.08.2015 US 201562211904 P; 31.08.2015 US 201562211922 P
(43) Date of publication of application: 11.07.2018
(73) Proprietor: CorMedix Inc., Berkeley Heights, NJ 07922 (US)
(72) Inventor: DILUCCIO, Robert, Viera, FL 32940 (US); REIDENBERG, Bruce, Rye, NY 10580 (US); MILBY, Randy, Long Valley, NJ 07853 (US)
(74) Representative: Müller, Thomas
(86) International application number: PCT/US2016/049656
(87) International publication number: WO 2017/040630

(56) References cited:
- EP-A2- 2 314 326
- WO-A1-2014/070796
- WO-A1-2015/052154
- DE-A1-102011 077 393
- US-A1- 2006 083 781
- US-A1- 2012 165 257
- US-A1- 2012 294 898
- US-A1- 2014 227 240
- HUYNH N T ET AL: "Lipid nanocapsules: A new platform for nanomedicine", INTERNATIONAL JOURNAL OF PHARMACEUTICS, ELSEVIER, NL, vol. 379, no. 2, 11 September 2009 (2009-09-11), pages 201-209, XP026519349, ISSN: 0378-5173, DOI: 10.1016/J.IJPHARM.2009.04.026 [retrieved on 2009-05-03]

## Description

### Field Of The Invention

This invention relates generally to compositions for treating joints, and more particularly to novel compositions for treating joints while maintaining an antimicrobial environment.

### Background Of The Invention

Osteoarthritis (OA), the most common form of arthritis, is a type of arthritis that is characterized by degenerative and/or abnormal changes in the bone, cartilage and/or synovium of joints. Osteoarthritis is often characterized by a progressive "wearing down" of opposing joint surfaces, and is sometimes accompanied by inflammation resulting in pain, swelling and/or stiffness for the patient. Osteoarthritis can occur in a joint following trauma to the joint, an infection of the joint or simply as a result of aging. Some evidence also suggests that abnormal anatomy may contribute to early development of osteoarthritis.

Osteoarthritis is typically treated by a combination of exercise or physical therapy, lifestyle modification and analgesics. Acetaminophen is typically the first analgesic used in the treatment of osteoarthritis. For mild to moderate symptoms, the effectiveness of acetaminophen is similar to the effectiveness of non-steroidal anti-inflammatory drugs (NSAIDs) such as ibuprofen, however, for more severe symptoms, NSAIDs may be more effective. However, while more effective, NSAIDs are associated with greater side effects such as gastrointestinal bleeding, renal complications, etc. Another class of NSAIDs which may be used to treat the symptoms of osteoarthritis are COX-2 selective inhibitors (e.g., celecoxib) which are equally effective to NSAIDs but no safer in terms of side effects. There are also several NSAIDs available for topical use, including diclofenac. Typically, such topical NSAIDs have less systemic side effects than oral administration and at least some therapeutic effect. While opioid analgesics (e.g., morphine, fentanyl, etc.) may be used to provide pain relief, this benefit is outweighed by the drawbacks inherent in using opioid analgesics, including drug dependence - for this reason, opioid analgesics are not routinely used to treat osteoarthritis. Intra-articular steroid injections are also sometimes used in the treatment of osteoarthritis, and they are generally highly effective at providing pain relief. However, the durability of the pain relief provided by intra-articular steroid injections is generally limited to 4-6 weeks, and there can be adverse effects which may include collateral cartilage damage. If pain becomes debilitating, joint replacement surgery may be used to improve mobility and quality of life. There is no proven treatment to slow or reverse osteoarthritis.

For patients who do not get adequate pain relief from exercise or physical therapy, lifestyle modification and simple pain relievers like acetaminophen, intra-articular injections of hyaluronic acid (HA) can provide another treatment option to address symptomatic pain and delay the need for total joint replacement surgery. It has been recognized that the concentration of native hyaluronic acid is typically deficient in individuals suffering from osteoarthritis and, therefore, intra-articular injections of exogenous hyaluronic acid can help replenish these molecules and restore the viscoelastic properties of the synovial fluid of a joint (i.e., the property of the joint that is responsible for lubricating and cushioning joints). There is also evidence that hyaluronic acid has biological activity through binding to cell surface receptors and may play a role in mitigating inflammation. Regardless of the mechanism of action, pain relief is observed for about six months following a treatment course comprising intra-articular injection of hyaluronic acid into the joint. A treatment course utilizing hyaluronic acid can range from a single injection of a hyaluronic acid to 3 to 5 "weekly" injections of hyaluronic acid in order to attain durable pain relief.

DE 10 2011 077 393 A1 describes a composition used for treating inflammations in a knee joint. The composition comprises hyaluronic acid and taurolidine. Other types of compositions for tissue repair or treating joints are disclosed in US 2012/294898 A1 and in US 2012/0165257 A1.

There remains a need for improved methods and compositions for treating osteoarthritic joints, and to address the pain and structural degeneration associated with osteoarthritis.

### Summary Of The Invention

According to the present invention, there is provided a novel composition which comprises the features of independent claim 1.

More particularly, the present invention generally comprises the provision and use of a novel composition comprising a hydrogel matrix of hyaluronic acid (HA) combined with taurolidine. The hydrogel matrix of hyaluronic acid (HA) and taurolidine is introduced into a joint in order to treat a joint condition such as osteoarthritis.

In one preferred form of the invention, the novel composition comprises hyaluronic acid, taurolidine and a bone morphogenetic protein (BMP), where the BMP is precipitated and dispersed in a hydrogel matrix of hyaluronic acid and taurolidine. The novel composition preferably has a pH of at least about 3, preferably a pH in the range of about 3 to 8, and more preferably a pH in the range of about 5-7.5. Also described is a method of treating a joint condition (e.g., osteoarthritis) by injecting the novel composition into a patient (e.g., into a patient's joint) in order to treat the joint condition, with the BMP becoming solubilized and biologically active within the patient after injection.

In one form of the invention, the BMP can be a growth and differentiation factor (GDF) protein, e.g., growth and differentiation factor 5 (GDF-5), growth and differentiation factor 6 (GDF-6), growth and differentiation factor 7 (GDF-7), etc. In another form of the invention, the BMP can be BMP2 or BMP7.

In one form of the invention, BMP is present in the novel composition at a concentration in the range of about 5-2000 micrograms/ml, and more preferably in the range of about 5-500 micrograms/ml. Furthermore, the BMP can be in a liquid state, or a solid/lyophilized state, which then can be combined with the hyaluronic acid and taurolidine. When in a liquid state, the BMP can be solubilized in an acid solution, e.g., hydrochloric acid, with a pH of less than about 4.

In one form of the invention, the hyaluronic acid can have a molecular weight of at least about 500 kilodaltons (kDa), and more preferably the hyaluronic acid has a molecular weight of at least about 1 million daltons or more. In one form of the invention, the novel composition comprises hyaluronic acid at a concentration in the range of approximately 5 mg/ml to approximately 60 mg/ml, and more preferably between approximately 7 mg/ml and approximately 30 mg/ml. The hyaluronic acid can be present in a liquid state, or in a solid/lyophilized state, prior to combining with the taurolidine and BMP. When in a liquid state, the hyaluronic acid can be solubilized in water, saline or buffered solution, or any other diluents known in the art. The hyaluronic acid solution preferably has a pH in the range of about 5 to 9 prior to combination with the taurolidine and the BMP.

In one form of the invention, the novel composition comprises an injectable formulation that includes taurolidine and a bone morphogenetic protein (BMP) dispersed within a hydrogel matrix of hyaluronic acid (HA).

In one form of the invention, the novel composition is formed by combining a hydrogel matrix of hyaluronic acid (HA), taurolidine and a bone morphogenetic protein (BMP) and allowing the combination to form a mixture containing a precipitate of the BMP that is dispersed within the hydrogel matrix.

Also described is a kit having a first component comprising a hydrogel matrix of hyaluronic acid (HA) and taurolidine, the hydrogel matrix having a pH in the range of about 3 to 8, and, optionally, a second component comprising an amount of a bone morphogenetic protein (BMP) that is in precipitate form and capable of becoming solubilized and biologically active following delivery of the novel composition to the joint of a patient. The novel kit preferably also comprises a syringe for injecting a mixture of the first component and the second component into the joint of a patient. If desired, the syringe can comprise a first chamber containing the first component, a second chamber containing the second component, and a plunger configured to move the second component out of the second chamber and into the first chamber so as to mix the first component and the second component together prior to delivery into the joint.

The taurolidine serves to protect the environment of injection and serves as a safety measure with prophylactic benefits. Taurolidine (bis(1,1-dioxoperhydro-1,2,4-thiadiazinyl-4)-methane) is known to have antimicrobial and antilipopolysaccharide properties. Taurolidine is derived from the amino acid taurine. Taurolidine's immunomodulatory action is reported to be mediated by priming and activation of macrophages and polymorphonuclear leukocytes.

Taurolidine has been used to treat patients with peritonitis and has been used as an antiendoxic agent in patients with systemic inflammatory response syndrome. Taurolidine is a life-saving antimicrobial for severe abdominal sepsis and peritonitis. For severe surgical infections and use in surgical oncology, taurolidine is active against a wide range of microorganisms, including gram positive bacteria, gram negative bacteria, fungi, mycobacteria and also bacteria that are resistant to various antibiotics such as Methicillin-Resistant Staphylococcus Aureus (MRSA), Vancomycin-Intermediate Staphylococcus Aureus (VISA), Vancomycin-Resistant Staphylococcus Aureus (VRSA), Oxacillin-Resistant Staphylococcus Aureus (ORSA), Vancomycin-Resistant Enterococci (VRE), etc. Additionally, taurolidine demonstrates some anti-tumor properties, with positive results seen in early-stage clinical investigations using taurolidine to treat gastrointestinal malignancies and tumors of the central nervous system.

Taurolidine is also the active ingredient of some antimicrobial catheter lock solutions used for the prevention and treatment of catheter-related blood stream infections (CRBSIs) and is suitable for use in all catheter-based vascular access devices. Bacterial resistance against taurolidine has not been observed in various studies to date.

Taurolidine acts by a non-selective chemical reaction. In aqueous solution, the parent molecule taurolidine forms an equilibrium with taurultam and N-hydroxymethyl taurultam, with taurinamide being a downstream derivative.

The active moieties of taurolidine are N-methylol derivatives of taurultam and taurinamide, which react with the bacterial cell wall, the cell membrane, and the proteins of the cell membrane, as well as with the primary amino groups of endo- and exotoxins. Microbes are killed and the resulting toxins are inactivated; the destruction time *in vitro* is approximately 30 minutes.

Pro-inflammatory cytokines and enhanced TNF-α levels are reduced when taurolidine is used as a catheter lock solution.

Taurolidine decreases the adherence of bacteria and fungi to host cells by destroying the fimbriae and flagella, thereby preventing biofilm formation.

In one preferred form of the invention, there is provided a novel hydrogel which comprises a matrix material (i.e., hyaluronic acid) and taurolidine, and, optionally, a a BMP which may be in the form of a precipitate that is dispersed in the hydrogel.

In one particularly preferred form of the invention, the novel composition comprises two components which are packaged separately and then mixed at the time of use: (i) a first hydrogel of a matrix material (i.e., hyaluronic acid) and taurolidine, and (ii) a second hydrogel of a matrix material (i.e., hyaluronic acid) and a BMP which may be in the form of a precipitate that is dispersed in the second hydrogel.

The present invention also provides another means for delivering hyaluronic acid into a joint which reduces the possibility of infections stemming from the injection of the hyaluronic acid into the joint. The present invention comprises the provision and use of a specialized formulation comprising hyaluronic acid and lipophilic nanoparticles whose center is a saturated solution of the antimicrobial taurolidine. The components of the nanoparticles are degraded slowly and metabolized to release the taurolidine, CO₂ and water. The taurolidine spontaneously hydrolyzes to the active methylol moieties, which prevent infection.

### Brief Description Of The Drawings

These and other objects and features of the present invention will be more fully disclosed or rendered obvious by the following detailed description of the preferred embodiments of the invention, which is to be considered together with the accompanying drawings wherein like numbers refer to like parts, and further wherein:
Fig. 1 is a schematic view showing one form of a mixing and delivery system for use with the novel compositions and methods of the present invention;
Fig. 2 is a schematic view showing another form of a mixing and delivery system for use with the novel compositions and methods of the present invention;
Fig. 3 is a schematic view of a matrix material (i.e., hyaluronic acid) having, in accordance with the present invention, taurolidine disposed thereon; and
Fig. 4 is a graph showing the complex viscosity of formulations with 3% taurolidine as a function of frequency sweep: the blue trace corresponds to LMW hyaluronic acid, the green trace corresponds to MMW hyaluronic acid, and the red trace corresponds to HMW hyaluronic acid.

### Detailed Description Of The Invention

The present invention generally comprises the provision and use of a novel composition for treating joint conditions such as osteoarthritis.

More particularly, in one preferred form of the invention, there is provided a novel hydrogel which comprises a matrix material (i.e., hyaluronic acid) and taurolidine, and, optionally, a BMP which may be in the form of a precipitate that is dispersed in the hydrogel.

In one particularly preferred form of the invention, the novel hydrogel comprises two components which are packaged separately and then mixed at the time of use: (i) a first hydrogel of a matrix material (i.e., hyaluronic acid) and taurolidine, and (ii) a second hydrogel of a matrix material (i.e., hyaluronic acid) and BMP, where the BMP is in the form of a precipitate that is dispersed in the second hydrogel. Various BMPs are suitable for use in the composition, as will hereinafter be discussed. By way of example but not limitation, suitable BMPs include a growth and differentiation factor (GDF), such as GDF-5, GDF-6, and GDF-7, and bone morphogenetic proteins (BMPs), such as BMP2 and BMP7.

The novel composition can be formulated as an injectable formulation that has a pH of at least about 3, and the pH can be in the range of about 3 to 8, more preferably the pH can be in the range of 4 to 7.5, even more preferably the pH can be in the range of 5 to 7.5.

The novel composition of the present invention can be used to treat a joint condition (e.g., osteoarthritis) by administering the composition to a patient, e.g., by injection into the body of the patient (e.g., by injection into a joint) as will hereinafter be discussed.

### Hyaluronic Acid

Hyaluronic acid (HA) can have various formulations and can be provided at various concentrations and molecular weights. The terms "hyaluronic acid," "hyaluronan," and "HA" are used interchangeably herein to refer to hyaluronic acids or salts of hyaluronic acid, such as the sodium, potassium, magnesium, and calcium salts of hyaluronic acid, among others. These terms are also intended to include not only elemental hyaluronic acid, but also hyaluronic acid with other trace components or in various compositions with other components. The terms "hyaluronic acid," "hyaluronan," and "HA" also encompass chemical, polymeric and/or cross-linked derivatives of hyaluronic acid. Examples of chemical modifications which may be made to hyaluronic acid include any reaction of an agent with the four reactive groups of hyaluronic acid, namely the acetamido, carboxyl, hydroxyl, and the reducing end. The hyaluronic acid used in the novel composition of the present invention is intended to include natural formulations, synthetic formulations, or combinations thereof. The hyaluronic acid can be provided in liquid or solid formulations, and the hyaluronic acid can be in pure liquid form or in a solvent at various concentrations.

Hyaluronic acid is a glycosaminoglycan (GAG), and, in particular, hyaluronic acid is an unbranched polysaccharide made up of alternating glucuronic acid and N-acetyl glucosamine units. Hyaluronic acid is a viscoelastic material that is also found in the extracellular matrix of cartilage bound to collagen. In particular, hyaluronic acid is an important building component of aggregated proteoglycans which impart resilient characteristics of articular cartilage. Hyaluronic acid not only helps keep the cartilage that cushions joints strong and flexible, but it also helps increase supplies of joint-lubricating synovial fluid. Hyaluronic acid abnormalities are a common "thread" in connective tissue disorders. Hyaluronic acid can thus be used to prevent, treat, or aid in the surgical repair of connective tissue disorders.

Hyaluronic acid can be used in the compositions of the present invention at various molecular weights. Since hyaluronic acid is a polymeric molecule, the hyaluronic acid component can exhibit a range of molecular weights, and almost any average of modal molecular weight formulation of hyaluronic acid can be used in the compositions and methods of the present invention, including Low Molecular Weight ("LWM") Hyaluronan (about 500 to 700 kilodaltons (kDa), Medium Molecular Weight ("MMW") Hyaluronan (700-1000 kDa), and High Molecular Weight ("HMW") Hyaluronan (1.0-4.0 million daltons (MDa)). In certain exemplary embodiments, the hyaluronic acid has a molecular weight of at least approximately 500 kDa, and more preferably the hyaluronic acid is a High Molecular Weight ("HWM") hyaluronic acid having a molecular weight of at least about 1 MDa. The molecular weight can be anywhere from 500 to 4000 kDa or more, or any range derivable therein. It is expected that chemically modified hyaluronic acid could have very different molecular weights than described above. A cross-linked hyaluronic acid can likewise have much higher molecular weight than noted above. Regardless, these materials may also be used with the present invention.

Solvents that can be used to solubilize hyaluronic acid include, but are not limited to, water, saline or other salt solutions, buffer solutions (e.g., phosphate buffered saline, histidine, lactate, succinate, glycine, glutamate, dextrose, glycerol, etc.) as well as combinations thereof.

The novel compositions of the present invention can include various other joint treatment agents or excipients, e.g., amino acids, proteins, saccharides, di-saccharides, poly-saccharides, nucleic acids, buffers, surfactants and mixtures thereof, steroids, anti-inflammatory agents, non-steroidal anti-inflammatory agents, analgesics, cells, stabilizers, antibiotics, antimicrobial agents, anti-inflammatory agents, growth factors, growth factor fragments, small-molecule wound healing stimulants, hormones, cytokines, peptides, antibodies, enzymes, isolated cells, platelets, immunosuppressants, nucleic acids, analgesics, cell types, viruses, virus particles, and combinations thereof.

The concentration of hyaluronic acid which is present in the novel composition of the present invention can also vary, but in a preferred form of the present invention, hyaluronic acid is provided at a pharmaceutically effective amount. In a preferred form of the present invention, the hyaluronic acid has a concentration of at least approximately 5 mg/ml, and more preferably at least approximately 7 mg/ml, and more preferably at least approximately 10 mg/ml, and more preferably at least approximately 15 mg/ml, and in some embodiments the concentration can be at least approximately 20 mg/ml. Suitable concentrations of hyaluronic acid include approximately 5 mg/ml to approximately 60 mg/ml or more or any range derivable therein.

In one embodiment of the present invention, the composition comprises hyaluronic acid having a high molecular weight (1 to 4 MDa) having a concentration in the range of about 7-40 mg/ml. One such product is Orthovisc™ manufactured by Anika Therapeutics, Inc. of Bedford, Mass. Orthovisc™ is a sterile, non-pyrogenic, clear, viscoelastic solution of hyaluronan. Orthovisc™ consists of high molecular weight (1.0-2.9 MDa), ultra-pure natural hyaluronan dissolved in physiological saline and having a nominal concentration of 15 mg/ml. Orthovisc™ is isolated through bacterial fermentation. One skilled in the art will recognize that there are companies such as Shiseido, Lifecore and Novozyme which can produce high molecular weight hyaluronic acid through a bacterial fermentation process. Another example of a hyaluronic acid product available in the United States with these characteristics is Euflexxa™.

### Taurolidine

While hyaluronic acid alone can be effective to treat joint conditions, hyaluronic acid combined with taurolidine provides a prophylactic effect and protection against a wide variety of microorganisms.

Taurolidine (bis(1,1-dioxoperhydro-1,2,4-thiadiazinyl-4)-methane) is known to have antimicrobial and antilipopolysaccharide properties. Taurolidine is derived from the amino acid taurine. Taurolidine's immunomodulatory action is reported to be mediated by priming and activation of macrophages and polymorphonuclear leukocytes.

Taurolidine has been used to treat patients with peritonitis and as an antiendoxic agent in patients with systemic inflammatory response syndrome. Taurolidine is a life-saving antimicrobial for severe abdominal sepsis and peritonitis. For severe surgical infections and use in surgical oncology, Taurolidine is active against a wide range of micro-organisms that include gram positive bacteria, gram negative bacteria, fungi, mycobacteria and also bacteria that are resistant to various antibiotics such as MRSA, VISA, VRSA, ORSA, VRE, etc. Additionally, taurolidine demonstrates some anti-tumor properties, with positive results seen in early-stage clinical investigations using the drug to treat gastrointestinal malignancies and tumors of the central nervous system.

Taurolidine is the active ingredient of antimicrobial catheter lock solutions for the prevention and treatment of catheter-related blood stream infections (CRBSIs) and is suitable for use in all catheter-based vascular access devices. Bacterial resistance against taurolidine has never been observed in various studies.

Taurolidine acts by a non-selective chemical reaction. In aqueous solution, the parent molecule taurolidine forms an equilibrium with taurultam and N-hydroxymethyl taurultam, with taurinamide being a downstream derivative.

The active moieties of taurolidine are N-methylol derivatives of taurultam and taurinamide, which react with the bacterial cell wall, cell membrane, and the proteins of the cell membrane, as well as with the primary amino groups of endo- and exotoxins. Microbes are killed and the resulting toxins are inactivated; the destruction time *in vitro* is 30 minutes. Pro-inflammatory cytokines and enhanced TNF-α levels are reduced when used as a catheter lock solution.

Taurolidine decreases the adherence of bacteria and fungi to host cells by destructing the fimbriae and flagella and thus prevent biofilm formation.

### Bone Morphogenetic Proteins

Another beneficial ingredient that can be added to the hyaluronic acid along with the taurolidine is a growth factor. In particular, Bone Morphogenetic Proteins (BMPs) can help bone and cartilage regeneration by effecting chondrogenesis. The present invention addresses the difficulty in administering hyaluronic acid in combination with BMPs since BMPs are generally not soluble at neutral pHs. BMPs are generally soluble in acidic solutions, however, it is not desirable to have a hyaluronic acid formulation at a sufficiently low pH to solubilize the BMP because hyaluronic acid is not stable at low pH and will degrade over time. However, a composition that combines BMP and hyaluronic acid and is maintained at a neutral/slightly acidic pH can ensure that the BMP remains stable during and after the precipitation process and demonstrates biological activity following precipitation and subsequent injection into a patient. Having a near-neutral pH formulation is also desirable from the perspective of the patient because there could be discomfort to the patient who receives injections of such acidic solutions. Thus, it has been discovered that the use of solid BMPs dispersed in solution combined with hyaluronic acid provides substantial benefits over hyaluronic acid alone. In particular, although the BMPs precipitate when combined with hyaluronic acid at or near neutral pH, the BMPs are able to resolubilize and become biologically active after injection into a patient's body. It is believed that the BMPs are not biologically active (or they have reduced biological activity) in their solid, precipitated form in hyaluronic acid, however, upon resolubilization after injection into a patient's body, the BMPs regain their biological activity and/or become more biologically active than in their solid, precipitated form.

The term "bone morphogenetic proteins" as used herein embraces the class of proteins typified by representatives of the TGF-β family subclass of true tissue morphogens. The BMPs that are useful can include, but are not limited to, growth and differentiation factors in both monomeric and dimeric forms (e.g., GDF-5, GDF-6 and GDF-7) and bone morphogenetic proteins (such as BMP2 and BMP7).

All members of this class of proteins share common structural features, including a carboxy terminal active domain, and are approximately 97-106 amino acids in mature length. They are translated as precursor proteins consisting of a prodomain, which is released proteolytically by members of the subtilisin-like proprotein convertase family, which is important in activating signaling that is conferred through the mature domain. All members of this class of proteins share a highly conserved pattern of cysteine residues that create three intramolecular disulfide bonds and one intermolecular disulfide bond. The active form can be either a disulfide-bonded homodimer of a single family member or a heterodimer of two different members. (See Massague Annu Rev. Cell Biol. 6:957 (1990); Sampath, et al. J. Biol. Chem. 265:13198 (1990); Ozkaynak et al. EMBO J. 9:2085-93 (1990); Wharton, et al. PNAS 88:9214-18 (1991); Celeste et al. PNAS 87:9843-47 (1990); Lyons et al. PNAS 86:4554-58 (1989), U.S. Pat. No. 5,011,691, and U.S. Pat. No. 5,266,683).

Osteogenic BMPs were initially identified by their ability to induce ectopic endochondral bone formation. (See Cheng et al. "Osteogenic activity of the fourteen types of human bone morphogenetic proteins" J. Bone Joint Surg. Am. 85A: 1544-52 (2003)). In particular, BMP2 and BMP7 play an important role in the development of bone and cartilage. BMP2 has been shown to stimulate the production of bone. BMP7 also plays a key role in the transformation of mesenchymal cells into bone and cartilage.

Growth/differentiation factors (GDF-1 to GDF-15) are initially synthesized as larger precursor proteins which subsequently undergo proteolytic cleavage at a cluster of basic residues approximately 110-140 amino acids from the C-terminus, thus releasing the C-terminal mature protein parts from the N-terminal prodomain. The mature polypeptides are structurally related and contain a conserved bioactive domain comprising six or seven canonical cysteine residues which is responsible for the characteristic three-dimensional "cysteine-knot" motif of these proteins. The mature proteins contain seven conserved cysteine residues that are assembled into active secreted homodimers. GDF dimers are disulfide-linked with the exception of GDF-3 and GDF-9. It will be appreciated by one skilled in the art that the term "GDF" is used interchangeably with "rhGDF."

GDF-5 is a morphogen which has been shown to promote cell proliferation, differentiation and/or tissue formation in several tissues. The protein is also known as morphogenetic protein MP52, bone morphogenetic protein BMP-14, and cartilage-derived morphogenetic protein-1 (CDMP-1). GDF-5 is closely related to GDF-6 and GDF-7, all of which can be used according to the present invention in combination with hyaluronic acid and taurolidine. These three proteins form a distinct subgroup of the TGF-β superfamily, thus displaying comparable biological properties and an extraordinary high degree of amino acid sequence identity. It has repeatedly been demonstrated that members of the GDF-5/GDF-6/GDF-7 subgroup are primarily important inducers and regulators of bone and cartilage as well as tendon and ligament.

Native GDF-5 proteins are homodimeric molecules and act mainly through interaction with specific receptor complexes which are composed of type I and type II serine/threonine receptor kinases. The receptor kinases subsequently activate SMAD proteins, which then propagate the signals into the nucleus to regulate target gene expression.

Biological molecules (biomolecules), such as BMPs, have three-dimensional structure or conformation, and rely on this structure for their biological activity and properties. Exposing these biomolecules to various environments such as variations in pH, temperature, solvents, osmolality, etc. can irreversibly change or denature the conformational state of the biomolecule, rendering it biologically inactive.

The chemistry and the three dimensional structure of each BMP family member impacts the solubility of the protein in an aqueous environment. BMP-2 is readily soluble at concentrations greater than 1 mg/ml when the pH is below 6, and above pH 6 the solubility can be increased by the addition of 1 M NaCl, 30% isopropanol, or 0.1 mM heparin (Ruppert, et al Eur J Biochem 237, 295-302 (1996)). The solubility of BMP-7/OP-1 is also limited at neutral pH. The solubility of GDF-5 is much more limited than that of BMP-2 or of BMP-7, and GDF-5 is nearly insoluble in physiological pH ranges and buffers. GDF-5 is only soluble in water at pH 2 to 4 (Honda, et al, Journal of Bioscience and Bioengineering 89(6), 582-589 (2000)). GDF-5 is soluble at an alkaline pH of about 9.5 to 12.0, however proteins degrade quickly under these conditions and thus acidic conditions have typically been used for preparation of GDF-5 protein. Solubility of these proteins are not only controlled by pH but are also affected by the salt concentrations or other active ingredients in the solution. For example, if there is an active ingredient in solution that the protein will bind to, it can cause insolubility of the protein.

Growth factors, such as BMPs, have been combined with other components. Due to the fact that solubility of most BMP's is limited at neutral pH, as noted above, BMP's are likely to precipitate out of solution in response to combination with neutral solutions, such as soluble hyaluronic acid. Many efforts have been made to prevent the precipitation and/or increase the maintenance of a bioactive BMP when combined with such components. However, no combinations have been previously disclosed for the utilization of a solid form of BMP, either precipitate or lyophilized, in a hyaluronic acid solution.

The novel compositions and methods of the present invention combine a solid or lyophilized formulation of BMP, such as rhGDF-5, with a liquid formulation of hyaluronic acid. Also in accordance with the present invention, the mixture of a solid or lyophilized formulation of BMP, such as rhGDF-5, with a liquid formulation of hyaluronic acid may further comprise, or be mixed with, taurolidine.

In another form of the present invention, novel compositions and methods are disclosed that form a mixture by combining a solution of BMP with a solution of hyaluronic acid, wherein the BMP forms a precipitate upon such a combination. And, in accordance with the present invention, the mixture of a solution of BMP with a solution of hyaluronic acid may further comprise, or be mixed with, taurolidine.

In accordance with the present invention, these components (i.e., solubilized hyaluronic acid, precipitated BMP and taurolidine) form a mixture that is subsequently administered to a patient (e.g., via intra-articular injection).

As noted above, the three dimensional conformational state of the BMP biomolecule is crucial for the activity of proteins including growth factors. The act of aggregating or precipitating such a biomolecule in solution can disturb the three dimensional structure, leading to loss of activity of the protein. Therefore, precautions are taken in order to prevent changes in the conformation or structure of the proteins because these changes can be irreversible.

The term "precipitation" as used herein refers to the formation of an insoluble protein in the solution. In contrast to known examples of drugs that are delivered as a suspension (due to the fact that the carrier, e.g., a mineral, ceramic, metal or polymeric, is insoluble rather than the active protein), an aspect of the present invention is the active precipitation of the protein (e.g., BMP) immediately prior to delivery of the mixture to the patient.

When in a liquid formulation, the BMP can be provided in water, saline, an acid solution (e.g., hydrochloric acid, acetic acid, benzoic acid, etc.), or another solvent suitable for solubilization of the BMP.

The concentration of the BMP present in the mixture can also vary, but in a preferred form of the present invention, BMP is provided in a pharmaceutically effective amount. By way of example but not limitation, the BMP may have a concentration of at least approximately 0.1 micrograms/ml, and more preferably at least approximately 5 micrograms/ml, and more preferably at least approximately 50 micrograms/ml, and more preferably at least approximately 200 micrograms/ml, and in some embodiments the concentration can be at least approximately 500 micrograms/ml. Suitable concentrations of BMP include approximately 5 micrograms/ml to 2000 micrograms/ml or more, or any range derivable therein.

### Lyophilization

It is also possible to lyophilize one or more of the components present in the novel composition of the present invention, and the one or more components can be lyophilized using various techniques known in the art. Lyophilization is a dehydration process that is typically used to preserve a perishable material - it works by freezing the material and then reducing the surrounding pressure and adding enough heat to allow the frozen water in the material to sublime directly from the solid phase to the gas phase. Standard lyophilization techniques known in the art can be used to lyophilize any one or more of the components of the novel mixture of the present invention. In an exemplary embodiment, at least the one or more BMPs are lyophilized.

Prior to lyophilization, various solvents can be used to form an aqueous mixture containing the component(s) to be lyophilized. In one form of the invention, an aqueous mixture is prepared by combining water with one or more of the components of the novel composition. The component(s) can be present within the mixture at various amounts, for example, rhGDF-5 may be present in the range of about 0.05 mg/mL to 10 mg/ml. In one form of the invention, the composition is filter sterilized, such as with a 0.2 micron filter, prior to lyophilization.

In another form of the invention, the component(s) of the novel composition can be lyophilized using the following cycle:
Freezing: From ambient temperature to 5 degrees C in 15 minutes. Hold at 5 degrees C for 100 minutes. Reduce to -45 degrees C in 50 minutes. Hold at -45 degrees C for 180 minutes.
Primary Drying: Set pressure at 50 mTorr (1 Torr is equal to 133 Pa). Shelf Up to -15 degrees C in 175 minutes. Hold at -15 degrees C for 2300 minutes.
Secondary Drying: Set pressure at 75 mTorr. Shelf Up to 25 degrees C in 200 minutes. Hold for 900 minutes.
Cycle End: Backfill with nitrogen to about 730 Torr, capping and crimping.

Variations to the foregoing temperatures, times and settings can be made in accordance with practices used by a person skilled in the art. Variations may include, but are not limited to, cycling temperatures for the freezing cycle, drying temperatures and end cycles. Variations may also include differences in holding times for the freezing, drying and capping/crimping cycles. Variations may also include differences in set pressures for the drying cycles and capping/crimping cycles. In addition, the number of drying cycles may be increased or decreased depending the apparatus used and/or component(s) to be lyophilized.

The addition of a buffering agent can provide for improved solubility and stability of the BMP in lyophilized formulations. Biocompatible buffering agents known in the art can be used, such as glycine; sodium, potassium, or calcium salts of acetate; sodium, potassium, or calcium salts of citrate; sodium, potassium, or calcium salts of lactate; and sodium or potassium salts of phosphate, including mono-basic phosphate, di-basic phosphate, tri-basic phosphate and mixtures thereof. The buffering agents can, additionally, have sugar added to the composition to function as a bulking agent. The pH preferably can be controlled within about 2.0 to about 5.0 pH units, and more preferably within about 2.5 to about 3.5 pH units.

### Formulations

In one preferred form of the present invention, the components of the novel composition (i.e., hyaluronic acid, taurolidine and BMP) are configured to be combined intraoperatively, i.e., immediately before or during an operation (e.g., an intra-articular injection). The components, when combined, can form a resulting composition or mixture having each component present in the resulting composition at various amounts. The amount of each component in the resulting composition can vary, but in an exemplary embodiment, the mixing ratio between hyaluronic acid and BMP can have a weight ratio of hyaluronic acid to BMP in the range of about 1:0.001 to about 1:0.3, and more preferably at a ratio in the range of about 1:0.005 to about 5:1, and the weight percentage of the taurolidine can be varied appreciably and can be anywhere near to or less than 2%. In other embodiments, a range of ratios, or any range derivable therein, between about 1:0.005 to about 5:1 of hyaluronic acid to BMP is envisioned, and the weight percentage of the taurolidine can be near to or less than 2%. Alternatively, compositions can include about 1% to about 75% or more by weight of each of the individual components, such as hyaluronic acid and BMP, in the total composition, alternatively about 2.5% to 70% or more by weight of hyaluronic acid and BMP in the total composition, and the weight percentage of the taurolidine can be near to or less than 2%. In an exemplary embodiment, the amount of hyaluronic acid present in the novel composition is about 1-4% by weight of the total composition, and the amount of BMP present in the novel composition is no more than about 2% by weight of the total composition, and the amount of taurolidine present in the novel composition can be anywhere near to or less than 2%.

Solvents that can be used to solubilize one or more of the components of the novel composition include, for example, water, acidic solvents, hydrochloric acid, acetic acid, benzoic acid, phosphate buffered saline, dextrose, glycerol, ethanol and the like, as well as combinations thereof. Solvents that can be used to solubilize hyaluronic acid can include, but are not limited to, water, saline or other salt solutions, buffer solutions such as phosphate buffered saline, histidine, lactate, succinate, glycine and glutamate, dextrose, glycerol, etc., as well as combinations thereof. Solvents that can be used to solubilize the BMP can include, but are not limited to, water, saline, hydrochloric acid, acetic acid, benzoic acid, acidic solvent, etc. The novel composition of the present invention can also include other components, such as dispersion media, coatings, antibacterial and antifungal agents, isotonic and absorption delaying agents, and the like that are physiologically compatible. Isotonic agents include, but are not limited to, sugars, polyalcohols such as mannitol, sorbitol, or sodium chloride, etc. The novel composition can also include minor amounts of auxiliary substances such as wetting or emulsifying agents, preservatives or buffers, which enhance the shelf life or effectiveness of the novel composition.

The components and/or the resulting novel composition can be sterilized prior to use using various techniques known in the art. Sterile injectable mixtures can be prepared by incorporating the active compound(s) in a therapeutically effective or beneficial amount in an appropriate solvent with one or a combination of ingredients, as required, followed by filtered sterilization. Generally, dispersions are prepared by incorporating compound(s), such as hyaluronic acid or BMP, into a sterile vehicle which contains a basic dispersion medium and any required other ingredients. In the case of sterile powders for the preparation of sterile injectable mixtures, some methods can include preparation of vacuum-dried and freeze-dried components which yield a powder of the composition plus any additional desired ingredients from a previously sterile-filtered mixture thereof.

The novel composition of the present invention can be incorporated into pharmaceutical compositions which are suitable for administration to a patient. Typically, the pharmaceutical composition comprises hyaluronic acid, taurolidine, at least one BMP and a pharmaceutically acceptable carrier. As used herein, the term "pharmaceutically acceptable carrier" includes any and all solvents, dispersion media, coatings, antibacterial and antifungal agents, isotonic and absorption delaying agents, and the like that are physiologically compatible. By way of example but not limitation, pharmaceutically acceptable carriers include water, hydrochloric acid, acetic acid, benzoic acid, acidic solvent, saline, phosphate buffered saline, dextrose, glycerol, ethanol, etc., as well as combinations thereof. In many cases, it will be preferable to include isotonic agents, e.g., sugars, polyalcohols such as mannitol, sorbitol, or sodium chloride, etc. in the novel composition of the present invention. Pharmaceutically acceptable carriers may further comprise minor amounts of auxiliary substances such as wetting or emulsifying agents, preservatives or buffers which enhance the shelf life or effectiveness of the novel composition of the present invention. The novel composition of the present invention can be provided in various states. By way of example but not limitation, the novel composition of the present invention may be provided in a liquid, semisolid and/or solid dosage states, such as liquid solutions (e.g., injectable and infusible solutions), dispersions or suspensions, tablets, pills, and powders. The preferred form of the novel composition typically depends on the intended mode of administration and therapeutic application. In a preferred form of the invention, the novel composition is provided in the form of an injectable or infusible solution (i.e., such as is typically used for in vivo injection). The preferred mode of administration of the novel composition is parenteral (e.g., intra-articular, subcutaneous, intraperitoneal and intramuscular). In one form of the invention, the novel composition can be administered by infusion or injection directly into the target area, e.g., a joint. In another form of the invention, the novel composition can be administered by intramuscular or subcutaneous injection.

Sterile injectable solutions can be prepared by incorporating the hyaluronic acid, taurolidine and BMP in therapeutically effective or beneficial amounts in an appropriate solvent, followed by filtered sterilization. Generally, dispersions are prepared by incorporating the hyaluronic acid, taurolidine and BMP into a sterile vehicle which contains a basic dispersion medium. In the case of sterile powders for use in the preparation of sterile injectable solutions, the preferred methods of preparation are vacuum-drying and freeze-drying which yields a powder of one or more of the hyaluronic acid, taurolidine and BMP from a previously sterile-filtered solution thereof - the powder may thereafter be placed into solution (and, where appropriate, mixed with others of the hyaluronic acid, taurolidine and BMP so as to form the novel composition).

### Delivery Systems

The novel composition and method of the present invention also encompasses the provision and use of kits for treating articular disorders, e.g., osteoarthritis and other disorders of the joints. In one form of the invention, there is provided a kit comprising a first component comprising hyaluronic acid and taurolidine, and a second component comprising hyaluronic acid and at least one BMP. Both components can be contained in a single chamber, or in separate chambers, of a syringe for injecting a mixture of the first and second components into the patient. In one form of the invention, the BMP comprises lyophilized GDF-5. In another form of the invention, the BMP comprises lyophilized GDF-6 or GDF-7. In another form of the invention, the second component (i.e., hyaluronic acid and at least one BMP) comprises more than one lyophilized BMP, wherein the lyophilized BMPs are selected from the group consisting of BMP2, BMP7, GDF-5, GDF-6 and GDF-7.

In another form of the invention, there is provided a kit comprising a first component comprising hyaluronic acid plus taurolidine, and a second component comprising hyaluronic acid plus BMP. The first component preferably comprises about 2 ml of Orthovisc™, which contains about 30 mg of hyaluronan, 18 mg of sodium chloride, and up to about 2.0 mL of water for injection. The hyaluronic acid has a molecular weight in the range of about 1.0 to 4 MDa. The second component preferably comprises about 0.005 to 3 mg of solid BMP (which is preferably lyophilized using the protocol discussed above). Alternatively, the BMP of the second component need not be lyophilized and can instead be provided in the form of a solution before combination with the first component.

The components comprising the novel composition of the present invention may be stored separately in order to increase shelf-life. The individual components may be lyophilized (or in solid form) when disposed in one syringe (or cartridge), and may be combined with diluent when disposed in a second syringe (or cartridge). In one form of the invention, one of the components is provided in lyophilized form (or in solid form) and the second component is provided in a solution capable of being combined with the lyophilized/solid compound so as to yield the novel composition. By way of example but not limitation, a first component comprising lyophilized (or solid) taurolidine can be stored in a first chamber and a second component comprising solubilized hyaluronic acid plus BMP can be stored in a second chamber. By way of further example but not limitation, both components can be lyophilized (or in solid form) and housed in a single (or separate) chambers of a syringe/cartridge. If desired, one or both components can be lyophilized directly in the syringe or cartridge.

Pre-filled dual-chamber syringes and/or cartridges can also be utilized in order to mix the components of the novel composition together so as to form the novel composition. Pre-filled dual-chamber syringes also enable the sequential administration of two separate components of the novel composition (or two doses of the novel composition) with a single syringe push, thereby replacing two syringes with one. The benefits of a single delivery capability include increasing the speed and ease of drug administration; reducing risk of infection by reducing the number of connections (i.e., injection sites); lowering the risk of drug administration or sequence errors, and quicker delivery of compositions requiring combination prior to administration. In a preferred form of the present invention, there is provided a dual-chamber syringe which accommodates lyophilized, powder or liquid formulations of hyaluronic acid, taurolidine and BMP in the front chamber and diluents, saline or buffer in the rear chamber. When the contents of the rear chamber are pushed into the front chamber, the components can mix together so as to form the novel composition, which can then be injected into the patient.

Pre-filled syringes can contain the exact deliverable dose of desired compounds and diluents. The pre-filled syringes preferably contain volumes of about 0.1 ml to 10 ml or more.

The dual syringe and/or cartridge can take the form of side-by-side chambers with separate syringe plungers (i.e., one plunger for each chamber) that are depressed in order to mix the two components within a single chamber, or the dual syringe and/or cartridge may take the form of two linear chambers with a single plunger. The dual chamber syringe and/or cartridges can also have a stopper or connector disposed between the two chambers so as to serve as a barrier between the two chambers. The stopper or connector can be removed in order to allow mixing or combining of the components contained within the two chambers.

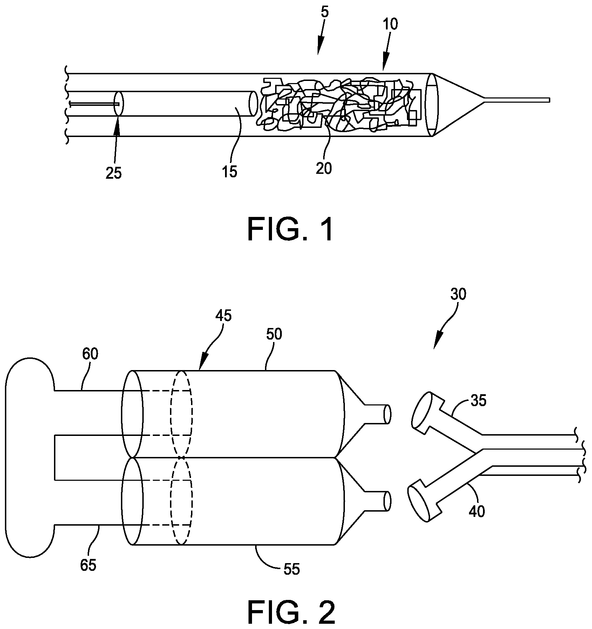

Fig. 1 shows a mixing and delivery system formed in accordance with the present invention. The mixing and delivery system is provided in the form of a dual chamber syringe 5. Dual chamber syringe 5 generally comprises a housing 10 having a proximal chamber 15 and a distal chamber 20 which are separated by a valve (not shown). A plunger 25 is moved within proximal chamber 15 and is configured to move material disposed within proximal chamber 15 into distal chamber 20, whereby to mix the two components (i.e., the component of proximal chamber 15 and the component of distal chamber 20). In one form of the invention, the first component comprises liquid hyaluronic acid and taurolidine disposed within proximal chamber 15 and the second component comprises one or more BMPs (with or without hyaluronic acid) disposed within the distal chamber 20. The plunger 25 can be advanced distally through proximal chamber 15, whereby to move the first component into distal chamber 20, whereby to mix the first component and the second component together. In another form of the invention, proximal chamber 15 comprises a solvent (e.g., water, saline, etc.) and distal chamber 20 comprises all of the components of the novel composition (i.e., hyaluronic acid, taurolidine and BMPs) in solid form. For example, distal chamber 20 can contain lyophilized or solid hyaluronic acid, taurolidine and one or more BMPs. Plunger 25 can be advanced distally through proximal chamber 15 so as to move the solvent from proximal chamber 15 into distal chamber 20, thereby solubilizing the components of the novel composition within distal chamber 20. Once all of the components of the novel composition are combined within distal chamber 20, the novel composition can be delivered to the patient, e.g., by attaching a needle to the distal end of the dual chamber syringe and injecting the novel composition into the patient.

Fig. 2 shows another mixing and delivery system 30, which is sold commercially under the trade name Merlin™ Mini-Dual Syringe. In this form of the invention, mixing and delivery system 30 comprises a fluid control assembly 35, 40 that is coupled between a syringe 45 and a vial (not shown). Syringe 45 comprises a first chamber 50 which can contain a liquid, e.g., a first component comprising liquid hyaluronic acid plus taurolidine, or a solvent, and comprises a second chamber 55 which can contain a solid, e.g., one or more BMPs (or, where the first component comprises a solvent, second chamber 22 may contain solid form hyaluronic acid, taurolidine and/or BMPs). Deployment of the plungers 60, 65 through the syringe 45 injects the components through the control assembly 35, 40 and into the vial (not shown), where the solid will be solubilized by the liquid. Once the components are fully solubilized, the vial can be inverted and the plungers 60, 65 can be retracted in order to draw the mixture back into the chambers 50, 55 in the syringe 45. The vial can then be removed from the system, and the mixture can be injected from the syringe through a needle into the patient.

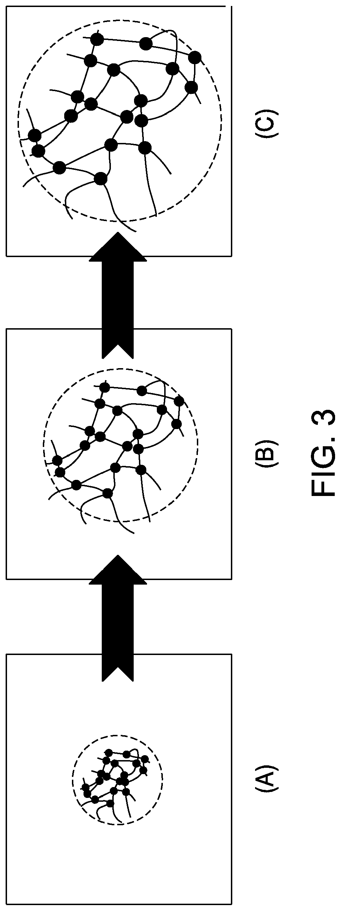

Fig. 3 is a schematic view representing a gel form of the novel composition of the present invention, with the gel being shown in its initial compressed state (A), gradually swelling (B), and arriving at an equilibrium state (C) when water is present. The gel is preferably deployed in its equilibrium state.

A person skilled in the art will appreciate that any dual chamber systems known in the art can be used, and that the chambers can be side-by-side chambers with separate syringe plungers that mix into a single chamber or linear chambers with a single plunger.

### Treatments

The novel composition of the present invention is preferably administered (for in vivo applications) parenterally by injection or by gradual perfusion over time. Administration may be intra-articular, intravenous, intraperitoneal, intramuscular, subcutaneous, intracavity, or transdermal. For in vitro studies, the hyaluronic acid, taurolidine and BMPs may be added or dissolved in an appropriate biologically acceptable buffer and added to a cell or tissue.

The hyaluronic acid, taurolidine and BMP can be co-administered or simultaneously administered in the same formulation or in two (or more) formulations that are combined via the same route. The hyaluronic acid, taurolidine and BMP components can be combined just prior to administration of the hyaluronic acid, taurolidine and BMP. The combination can occur within seconds, minutes, hours, days or weeks prior to the administration of the composition.

By way of example but not limitation, preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions and suspensions (including saline and buffered media). Parenteral vehicles include sodium chloride solutions, Ringer's dextrose, dextrose and sodium chloride, and lactated Ringer's intravenous vehicles which include fluid and nutrient replenishers, electrolyte replenishers (such as those based on Ringer's dextrose), and the like. Preservatives and other additives may also be present such as, for example, anti-microbials, anti-oxidants, chelating agents, growth factors and inert gases and the like.

Frequently used "carriers" or "auxiliaries" include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. Intravenous vehicles include fluid and nutrient replenishers. Preservatives include antimicrobials, anti-oxidants, chelating agents and inert gases. Other pharmaceutically acceptable carriers include aqueous solutions, non-toxic excipients, including salts, preservatives, buffers and the like, as described, for instance, in Remington's Pharmaceutical Sciences, 15th ed. Easton: Mack Publishing Co.: 1405-1412, 1461-1487, 1975 and The National Formulary XIV., 14th ed. Washington: American Pharmaceutical Association, 1975. The pH and exact concentration of the various components of the pharmaceutical composition are adjusted according to routine skills in the art. See Goodman and Gilman's The Pharmacological Basis for Therapeutics (7th ed.).

Examples of symptoms or diseases which the novel composition can be used to treat include, but are not limited to, articular disorders (such as arthritis caused by infections), injuries, allergies, metabolic disorders, etc., rheumatoids such as chronic rheumatoid arthritis, and systemic lupus erythematosus, articular disorders accompanied by gout, arthropathy such as osteoarthritis, internal derangement, hydrarthrosis, stiff neck, lumbago, etc. Varying the effects depending on the use of the composition or the types of diseases to be treated, the agent can exert desired prophylactic and alleviative effects, or even therapeutic effects on swelling, pain, inflammation, and destroying of articulations without seriously affecting living bodies. The composition for treating articular disorder can be used to prevent the onset of articulation disorders, as well as to improve, alleviate, and cure the symptoms after their onsets. The claimed compositions are suitable for treating a joint condition. Further disclosed are methods of treatment. However, the scope of the invention is defined by the claims. Any references in the description to methods of treatment refer to the compounds, pharmaceutical compositions and medicaments of the present invention for use in a method for treatment of the human (or animal) body by therapy (or for diagnosis). The methods of treatment can include directly injecting the compositions into the target area, such as a joint. Injections can be performed as often as daily, weekly, several times a week, bi-monthly, several times a month, monthly, or as often as needed as to provide relief of symptoms. For intra-articular use, from about 1 to about 40 mg/ml of hyaluronic acid, taurolidine and one or more BMPs per joint, depending on the size of the joint and severity of the condition, can be injected. The frequency of subsequent injections into a given joint are spaced to the time of recurrence of symptoms in the joint. Illustratively, dosage levels in humans of the composition can be: knee, about 0.001 to about 40 mg/ml of hyaluronic acid, taurolidine and one or more BMPs per joint injection; shoulder, about 0.001 to about 40 mg/ml of hyaluronic acid, taurolidine and one or more BMPs per joint injection; metacorpal or proximal intraphalangeal, about 0.001/ml to about 40 mg/ml of hyaluronic acid, taurolidine and one or more BMPs per joint injection; and elbow, about 1 to about 300 mg of hyaluronic acid, taurolidine and one or more BMPs per joint injection.

It will be understood, however, that the specific dosage level for any particular patient will depend upon a variety of factors including the activity of the specific compound employed, the age, body weight, general health, sex, diet, time of administration, route of administration, rate of excretion, drug combination and the severity of the particular disease undergoing therapy. The pharmaceutical compositions can be prepared and administered in dose units. Under certain circumstances, however, higher or lower dose units may be appropriate. The administration of the dose unit can be carried out both by single administration of the composition or administration can be performed in several smaller dose units and also by multiple administrations of subdivided doses at specific intervals.

In one embodiment, the medical condition is osteoarthritis (OA) and the composition is administered in a joint space, such as, for example, a knee, shoulder, temporo-mandibular and carpo-metacarpal joints, elbow, hip, wrist, ankle, and lumbar zygapophysial (facet) joints in the spine. The viscosupplementation may be accomplished via a single injection or multiple intra-articular injections administered over a period of weeks into the knee or other afflicted joints. For example, a human subject with knee OA may receive one, two, or three injections of about 2, 3, 4, 5, 6, 7, 8, 9, 10 ml or more per knee. For other joints, the administered volume can be adjusted based on the size on the joint.

It will be understood, however, that the specific dosage level for any particular patient will depend upon a variety of factors including the activity of the specific compound employed, the age, body weight, general health, sex, diet, time of administration, route of administration, rate of excretion, drug combination and the severity of the particular disease undergoing therapy.

### Examples

The following evaluations were done to assess the performance of gels comprising hyaluronic acid and taurolidine. Solution exposure of the hyaluronic acid/taurolidine hydrogels, immersed in early phase concentrations of 2 test microorganisms, were conducted. Additionally, the physical properties of the hydrogels were assessed to determine their shear/thixotropic properties.

Hyaluronic Acid Hydrogel Preparation. Formulations of taurolidine in aqueous solutions of hyaluronic acid, crosslinked with 1,4-butanediol diglycidyl ether (BDDE), were prepared. Three concentrations (1.5%, 3% and 6%) of taurolidine were formulated in aqueous solutions of crosslinked hyaluronic acid of three molecular weights: low molecular weight (LMW) 21-40 kDa, medium molecular weight (MMW) 310-450 kDa and high molecular weight (HMW) 750 kDa-1.0 MDa. Control formulations were prepared without the addition of taurolidine. The compositions of each formulation are given in Table 1 below.

**Composition of formulations. Formulations according to the claims comprise hyaluronic acid and nanoparticles comprising a taurolidine core surrounded by a lipophilic coating. Table 1**

| Sample | HA | HA [%] | Taurolidine [%] | BDDE [%] | BDDE/HA [] | pH |
|---|---|---|---|---|---|---|
| Controls (no taurolidine) | | | | | | |
| 13079-1 | LMW | 2.0 | 0 | 1.0 | 0.5 | 7.2 |
| 13079-2 | MMW | 2.0 | 0 | 0.9 | 0.5 | 7.0 |
| 13079-3 | HMW | 2.0 | 0 | 0.9 | 0.5 | 7.5 |

| 1.5 % taurolidine | | | | | | |
|---|---|---|---|---|---|---|
| 13079-4 | LMW | 2.0 | 1.5 | 0.9 | 0.5 | 8.0 |
| 13079-5 | MMW | 1.9 | 1.5 | 0.9 | 0.5 | 7.6 |
| 13079-6 | HMW | 1.9 | 1.5 | 0.9 | 0.5 | 7.9 |

| 3 % taurolidine | | | | | | |
|---|---|---|---|---|---|---|
| 13079-7 | LMW | 2.0 | 2.9 | 0.9 | 0.4 | 7.0 |
| 13079-8 | MMW | 2.0 | 3.0 | 0.9 | 0.5 | 7.3 |
| 13079-9 | HMW | 2.0 | 2.9 | 0.9 | 0.5 | 7.7 |

| 6 % taurolidine | | | | | | |
|---|---|---|---|---|---|---|
| 13079-10 | LMW | 2.0 | 6.1 | 1.0 | 0.5 | 7.3 |
| 13079-11 | MMW | 2.0 | 6.0 | 0.9 | 0.5 | 7.8 |
| 13079-12 | HMW | 2.0 | 6.1 | 1.0 | 0.5 | 7.7 |

Solution Exposure of Hyaluronic Acid to Living Cultures. The bacteria kills with taurolidine-loaded hydrogels placed in solutions with living microorganisms was demonstrated. In this study, two strains of bacteria, Pseudomonas aeruginosa (PAO1) and Stapylococcus aureus strain SA 113), were evaluated for total kill of each of the strains after 4 and 24 hours of exposure. 20 µl of early phase culture of each strain was placed in 980 µl of each hyaluronic acid/taurolidine formulation. Samples of remaining bacteria concentrations were determined for the samples after 4 and 24 hours of exposure. Four controls were prepared, one control without hyaluronic acid and three other controls with different molecular weights of hyaluronic acid (low, medium and high MW) (all without taurolidine). These were compared against the same 3 different molecular weights of hyaluronic acid which contained taurolidine, with each hyaluronic acid MW containing target concentrations of 1.5, 3, or 6% taurolidine by weight in the hyaluronic acid hydrogel matrix (Table 1). This resulted in a total of 12 hyaluronic acid hydrogels: 9 with taurolidine and 3 without taurolidine. These were also evaluated against a known antimicrobial, gentamicin. The results are shown in Tables 2 and 3 below.

**Remaining bacteria concentrations after 4 and 24 hour exposure to HA/taurolidine gels. Formulations according to the claims comprise hyaluronic acid and nanoparticles comprising a taurolidine core surrounded by a lipophilic coating. Table 2**

| Sample | PA01 (4hr) | PA01 (24hr) | SA113(4hr) | SA113(24hr) | | | | |
|---|---|---|---|---|---|---|---|---|
| Control (no HA) | 1.40E+08 | 1.40E+08 | 1.72E+08 | 1.72E+08 | STDV1 | STDV2 | STDV3 | STDV4 |
| 13079-1 | 7.63E+03 | 0.00E+00 | 0.00E+00 | 0.00E+00 | 1.07E+04 | 0.00E+00 | 0.00E+00 | 0.00E+00 |
| 13079-2 | 2.02E+06 | 0.00E+00 | 1.44E+05 | 0.00E+00 | 9.99E+05 | 1.67E+04 | 0.00E+00 | 0.00E+00 |
| 13079-3 | 2.84E+06 | 2.51E+05 | 2.24E+05 | 0.00E+00 | 7.31E+04 | 1.78E+05 | 2.87E+05 | 0.00E+00 |
| 13079-4 | 0.00E+00 | 0.00E+00 | 0.00E+00 | 0.00E+00 | 0.00E+00 | 0.00E+00 | 0.00E+00 | 0.00E+00 |
| 13079-5 | 0.00E+00 | 0.00E+00 | 0.00E+00 | 0.00E+00 | 0.00E+00 | 0.00E+00 | 0.00E+00 | 0.00E+00 |
| 13079-6 | 0.00E+00 | 0.00E+00 | 0.00E+00 | 0.00E+00 | 0.00E+00 | 0.00E+00 | 0.00E+00 | 0.00E+00 |
| 13079-7 | 0.00E+00 | 0.00E+00 | 0.00E+00 | 0.00E+00 | 0.00E+00 | 0.00E+00 | 0.00E+00 | 0.00E+00 |
| 13079-8 | 0.00E+00 | 0.00E+00 | 0.00E+00 | 0.00E+00 | 0.00E+00 | 0.00E+00 | 0.00E+00 | 0.00E+00 |
| 13079-9 | 0.00E+00 | 0.00E+00 | 0.00E+00 | 0.00E+00 | 0.00E+00 | 0.00E+00 | 0.00E+00 | 0.00E+00 |
| 13079-10 | 0.00E+00 | 0.00E+00 | 0.00E+00 | 0.00E+00 | 0.00E+00 | 0.00E+00 | 0.00E+00 | 0.00E+00 |
| 13079-11 | 0.00E+00 | 0.00E+00 | 0.00E+00 | 0.00E+00 | 0.00E+00 | 0.00E+00 | 0.00E+00 | 0.00E+00 |
| 13079-12 | 0.00E+00 | 0.00E+00 | 0.00E+00 | 0.00E+00 | 0.00E+00 | 0.00E+00 | 0.00E+00 | 0.00E+00 |
| Gentamicin | 0.00E+00 | 0.00E+00 | 3.77E+02 | 8.33E+00 | 0.00E+00 | 3.77E+01 | 0.00E+00 | 2.36E+00 |

**Complex viscosity for HA/taurolidine formulations at a frequency of 1 Hz All viscosities in Pascal seconds (Pa. s). Formulations according to the claims comprise hyaluronic acid and nanoparticles comprising a taurolidine core surrounded by a lipophilic coating. Table 3**

| Formulation | 0% taurolidine [Pa.s] | Formulation | 1.5 % taurolidine [Pa.s] | Formulation | 3% taurolidine [Pa.s] | Formulation | 6% taurolidine [Pa.s] |
|---|---|---|---|---|---|---|---|
| 13079-1 | 0.01 | 13079-4 | 0.01 | 13079-7 | 0.01 | 13079-10 | 0.01 |
| 13079-2 | 0.09 | 13079-5 | 0.13 | 13079-8 | 0.25 | 13079-11 | 0.55 |
| 13079-3 | 0.21 | 13079-6 | 0.40 | 13079-9 | 1.47 | 13079-12 | 6.68 |

As can be seen in the Table 2 above, all samples of hydrogels containing taurolidine provided total kill of both Pseudomonas aeruginosa (PAO1) and Stapylococcus aureus strain (SA 113) after only 4 hours of exposure as compared to the controls.

As indicated in Table 3 above, high MW versions of hyaluronic acid (HA) containing taurolidine had enhanced thixotropic properties by measure of complex viscosity, giving values of 0.21, 0.4 and 1.47 and 6.68 Pa seconds at taurolidine concentrations of 0, 1.5, 3, and 6%, respectively, which increased with taurolidine concentration.

Medium MW versions of hyaluronic acid containing taurolidine also exhibited thixotropic properties to a lesser degree with increasing taurolidine concentration, with values of 0.09, 0.13, 0.25, 0.55 Pa seconds at taurolidine concentrations of 0, 1.5, 3 and 6%, respectfully.

All the low MW hyaluronic acid (HA) gels containing taurolidine exhibited values of 0.01 Pa seconds for all the samples and did not exhibit thixotropic properties.

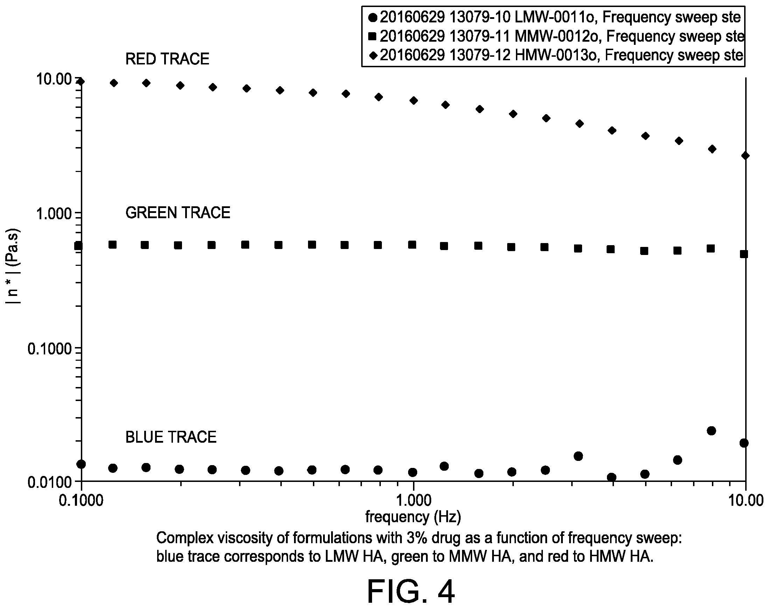

Fig. 4 illustrates the thixotropic nature of the gels and shows that the higher MW hyaluronic acid formulation with the taurolidine has enhanced thixotropic properties.

### Specialized Formulation Comprising Hyaluronic Acid And Lipophilic Nanoparticles Whose Center Is A Saturated Solution Of Taurolidine

As noted above, the intra-articular injection of hyaluronic acid (HA) into arthritic joints to improve mobility has become part of the standard of care. Current preventative treatment to avoid infection from the injection of the hyaluronic acid involves pre-sterilized, single use pre-loaded syringes. No antiseptic is added to the product and no preservative is added. The published information on the infection rate from intra-articular "viscosupplementation" is "very rare" but not quantified.

To this end, the novel composition described above, which comprises hyaluronic acid with taurolidine and BMP, provides an effective means for delivering hyaluronic acid into a joint which reduces the possibility of infections stemming from the injection of the hyaluronic acid into the joint.

The present invention also provides another means for delivering hyaluronic acid into a joint which reduces the possibility of infections stemming from the injection of the hyaluronic acid into the joint.

More particularly, the present invention comprises the provision and use of a specialized formulation comprising hyaluronic acid and lipophilic nanoparticles whose center is a saturated solution of the antimicrobial taurolidine. The components of the nanoparticles are degraded slowly and metabolized to release the taurolidine, CO₂ and water. The taurolidine spontaneously hydrolyzes to the active methylol moieties, which prevent infection.

In one form of the invention, there is provided a composition which comprises a carrier and lipid-coated nanoparticles comprising taurolidine. In one preferred form of the invention, the carrier comprises a hydrogel matrix which, when metabolized, provides a tissue scaffold and exposes the lipid-coated nanoparticles to bodily fluids. In one preferred form of the invention, the hydrogel matrix comprises hyaluronic acid. The lipid-coated nanoparticles comprise nanoparticles of taurolidine surrounded by lipophilic peptides such that when the lipid-coated nanoparticles are exposed to bodily fluids, the lipophilic peptides are metabolized to release the taurolidine. The taurolidine then spontaneously hydrolyzes to the active methylol moieties, which prevent infection. Significantly, the tissue scaffold provided by the metabolized hydrogel matrix helps maintain the taurolidine at the appropriate anatomical location as bone tissue grows into the tissue scaffold.

More particularly, taurolidine is a well-known antimicrobial with a published mechanism of action and antimicrobial spectrum. Taurolidine is unstable in circulation and therefore has not been successfully developed for systemic infections. Taurolidine has demonstrated efficacy in local application for peritonitis and for prevention of infection when infused as catheter-lock solution. These uses confirm in humans the broad spectrum anti-microbial activity seen with Taurolidine in vitro. Prevention of post-procedure intra-articular infection requires a broad-spectrum product with minor safety liabilities.

The use of taurolidine as an antiseptic/antimicrobial in intra-articular products is limited by its instability in aqueous media prior to injection into the body.

The present invention provides specialized formulations designed to maintain taurolidine stability prior to injection and not impair the hydrolysis of taurolidine to methylol active moieties. These formulations are based on lipid-coated nanoparticles within a hydrogel matrix (e.g., hyaluronic acid). The lipid coating needs to be degraded slowly into non-inflammatory breakdown products. For this reason, careful selection of lipophilic peptides or fatty acid esters is designed to ensure that the breakdown products of the lipid coating are metabolizable to CO₂ and water.

To this end, a saturated solution of taurolidine is mixed with the lipophilic components (either fatty acid esters or lipophilic peptides) and spray evaporated to form dry particles. The dry particles are suspended in the hyaluronic acid gel to make the final injectable product.

In one form of the invention, there is provided a composition which comprises a carrier and lipid-coated nanoparticles comprising taurolidine. In one preferred form of the invention, the carrier comprises a hydrogel matrix which, when metabolized, provides a tissue scaffold and exposes the lipid-coated nanoparticles to bodily fluids. In one preferred form of the invention, the hydrogel matrix comprises hyaluronic acid. The lipid-coated nanoparticles comprise nanoparticles of taurolidine surrounded by lipophilic peptides such that when the lipid-coated nanoparticles are exposed to bodily fluids, the lipophilic peptides are metabolized to release the taurolidine. The taurolidine then spontaneously hydrolyzes to the active methylol moieties, which prevent infection. Significantly, the tissue scaffold provided by the metabolized hydrogel matrix helps maintain the taurolidine at the appropriate anatomical location as bone tissue grows into the tissue scaffold.

## Claims

1. A composition for treating a joint condition, the composition comprising:
hyaluronic acid; and
nanoparticles comprising a taurolidine core surrounded by a lipophilic coating;
wherein the hyaluronic acid comprises a hydrogel, and wherein the hydrogel metabolizes to provide a tissue scaffold and expose the nanoparticles to bodily fluids

2. The composition according to claim 1,
wherein the hyaluronic acid has a concentration of at least approximately 5 mg/ml, preferably a concentration of at least approximately 7 mg/ml, preferably a concentration of at least approximately 10 mg/ml, preferably a concentration of at least approximately 15 mg/ml, preferably a concentration of at least approximately 20 mg/ml, preferably a concentration of at least approximately 60 mg/ml.

3. The composition according to claim 1,
wherein the hyaluronic acid has a molecular weight of at least about 500 kDa, preferably a molecular weight of at least about 1 MDa, preferably a molecular weight of about 1-4 MDa.

4. The composition according to claim 1,
wherein the taurolidine comprises approximately 6% or less of the composition by weight, preferably approximately 2% or less of the composition by weight.

5. The composition according to claim 1, further comprising at least one bone morphogenetic protein (BMP), wherein optionally the at least one BMP comprises one from the group consisting of BMP2 , BMP7, GDF-5, GDF-6, and GDF-7.

6. The composition according to claim 5,
wherein the BMP is at a concentration of at least approximately 0.1 µg/ml, preferably at a concentration of at least approximately 5 *µ*g/ml, preferably at a concentration of at least approximately 50 *µ*g/ml, preferably at a concentration of at least approximately 200 *µ*g/ml, preferably at a concentration of at least approximately 500 *µ*g/ml, preferably at a concentration of at least approximately 2000 *µ*g/ml.

7. The composition according to claim 5,
wherein the BMP is in solid form.

8. The composition according to claim 1,
wherein the tissue scaffold provided by the metabolized hydrogel helps maintain the taurolidine core at the appropriate anatomical location as bone tissue grows into the tissue scaffold.

9. The composition according to claim 1,
wherein the taurolidine core comprises a saturated solution of taurolidine.

10. The composition according to claim 1,
wherein the lipophilic coating comprises lipophilic peptides, wherein the lipophilic coating optionally comprises mixtures of mono-, di-, and tri-glycerides, or wherein the lipophilic coating optionally comprises at least one from the group consisting of valine, leucine, proline, phenylalanine and tryptophan, or combinations of the peptides.

11. The composition according to claim 1,
wherein, when the nanoparticles are exposed to bodily fluids, the lipophilic coating is metabolized to release the taurolidine core, and wherein optionally the taurolidine core spontaneously hydrolyzes to active methylol moieties when the lipophilic coating is metabolized.

## Patentansprüche

1. Zusammensetzung zur Behandlung einer Gelenkerkrankung, wobei die Zusammensetzung aufweist:
Hyaluronsäure; und
Nanopartikel, die einen Taurolidinkern aufweisen, der von einer lipophilen Beschichtung umgeben ist;
wobei die Hyaluronsäure ein Hydrogel aufweist, und wobei das Hydrogel metabolisiert, um ein Gewebegerüst bereitzustellen und die Nanopartikel Körperflüssigkeiten auszusetzen.

2. Zusammensetzung nach Anspruch 1, wobei die Hyaluronsäure eine Konzentration von mindestens ungefähr 5 mg/ml, vorzugsweise eine Konzentration von mindestens ungefähr 7 mg/ml, vorzugsweise eine Konzentration von mindestens ungefähr 10 mg/ml, vorzugsweise eine Konzentration von mindestens ungefähr 15 mg/ml, vorzugsweise eine Konzentration von mindestens ungefähr 20 mg/ml, vorzugsweise eine Konzentration von mindestens ungefähr 60 mg/ml aufweist

3. Zusammensetzung nach Anspruch 1, wobei die Hyaluronsäure ein Molekulargewicht von mindestens etwa 500 kDa, vorzugsweise ein Molekulargewicht von mindestens etwa 1 MDa, vorzugsweise ein Molekulargewicht von etwa 1-4 MDa aufweist.

4. Zusammensetzung nach Anspruch 1, wobei das Taurolidin ungefähr 6 Gew.-% oder weniger der Zusammensetzung aufweist, vorzugsweise ungefähr 2 Gew.-% der Zusammensetzung.

5. Zusammensetzung nach Anspruch 1, weiterhin aufweisend mindestens ein knochenmorphogenetisches Protein (BMP), wobei optional das mindestens eine BMP eines aus der Gruppe umfasst, die aus BMP2, BMP7, GDF-5, GDF-6 und GDF-7 besteht.

6. Zusammensetzung nach Anspruch 5, wobei das BMP eine Konzentration von mindestens ungefähr 0.1 µg/ml, vorzugsweise eine Konzentration von mindestens ungefähr 5 µg/ml, vorzugsweise eine Konzentration von mindestens ungefähr 50 µg/ml, vorzugsweise eine Konzentration von mindestens ungefähr 200 µg/ml, vorzugsweise eine Konzentration von mindestens ungefähr 500 µg/ml, vorzugsweise eine Konzentration von mindestens ungefähr 2000 µg/ml, hat.

7. Zusammensetzung nach Anspruch 5, wobei das BMP in fester Form vorliegt.

8. Zusammensetzung nach Anspruch 1, wobei das durch das metabolisierte Hydrogel bereitgestellte Gewebegerüst dazu beiträgt, den Taurolidinkern an der geeigneten anatomischen Stelle zu halten, wenn Knochengewebe in das Gewebegerüst hineinwächst.

9. Zusammensetzung nach Anspruch 1, wobei der Taurolidinkern eine gesättigte Lösung von Taurolidin aufweist.

10. Zusammensetzung nach Anspruch 1, wobei die lipophile Beschichtung lipophile Peptide aufweist, wobei die lipophile Beschichtung optional Gemische von Mono-, Di- und Tri-Glyceriden aufweist, oder wobei die lipophile Beschichtung optional mindestens eine aus der Gruppe bestehend aus Valin, Leucin, Prolin, Phenylalanin und Tryptophan, oder Kombinationen der Peptide aufweist.

11. Zusammensetzung nach Anspruch 1, wobei, wenn die Nanopartikel Körperflüssigkeiten ausgesetzt sind, die lipophile Beschichtung metabolisiert wird, um den Taurolidinkern freizusetzen, und wobei optional der Taurolidinkern spontan zu aktiven Methyloleinheiten hydrolysiert, wenn die lipophile Beschichtung metabolisiert wird.

## Revendications

1. Composition pour le traitement d'une affection articulaire, la composition comprenant :
de l'acide hyaluronique ; et
des nanoparticules comprenant un noyau de taurolidine entouré par un revêtement lipophile ;
dans laquelle l'acide hyaluronique comprend un hydrogel, et dans laquelle l'hydrogel se métabolise pour fournir un échafaudage tissulaire et exposer les nanoparticules aux fluides corporels.

2. Composition selon la revendication 1, dans laquelle l'acide hyaluronique a une concentration d'au moins approximativement 5 mg/ml, de préférence une concentration d'au moins approximativement 7 mg/ml, de préférence une concentration d'au moins approximativement 10 mg/ml, de préférence une concentration d'au moins approximativement 15 mg/ml, de préférence une concentration d'au moins approximativement 20 mg/ml, de préférence une concentration d'au moins approximativement 60 mg/ml.

3. Composition selon la revendication 1, dans laquelle l'acide hyaluronique a une masse moléculaire d'au moins environ 500 kDa, de préférence une masse moléculaire d'au moins environ 1 MDa, de préférence une masse moléculaire d'environ 1 à 4 MDa.

4. Composition selon la revendication 1, dans laquelle la taurolidine comprend approximativement 6 % ou moins de la composition en poids, de préférence approximativement 2 % ou moins de la composition en poids.

5. Composition selon la revendication 1, comprenant en outre au moins une protéine morphogénétique osseuse (BMP), dans laquelle facultativement l'au moins une BMP comprend l'une du groupe consistant en les BMP2, BMP7, GDF-5, GDF-6, et GDF-7.

6. Composition selon la revendication 5, dans laquelle la BMP est en une concentration d'au moins approximativement 0,1 µg/ml, de préférence en une concentration d'au moins approximativement 5 µg/ml, de préférence en une concentration d'au moins approximativement 50 µg/ml, de préférence en une concentration d'au moins approximativement 200 µg/ml, de préférence en une concentration d'au moins approximativement 500 µg/ml, de préférence en une concentration d'au moins approximativement 2 000 µg/ml.

7. Composition selon la revendication 5, dans laquelle la BMP est sous une forme solide.

8. Composition selon la revendication 1, dans laquelle l'échafaudage tissulaire fourni par l'hydrogel métabolisé aide à maintenir le noyau de taurolidine au niveau de l'emplacement anatomique approprié alors que le tissu osseux se développe dans l'échafaudage tissulaire.

9. Composition selon la revendication 1, dans laquelle le noyau de taurolidine comprend une solution saturée de taurolidine.

10. Composition selon la revendication 1, dans laquelle le revêtement lipophile comprend des peptides lipophiles, dans laquelle le revêtement lipophile comprend facultativement des mélanges de mono-, di-, et tri-glycérides, ou dans laquelle le revêtement lipophile comprend facultativement au moins l'un du groupe consistant en la valine, la leucine, la proline, la phénylalanine et le tryptophane, ou des combinaisons des peptides.

11. Composition selon la revendication 1, dans laquelle, lorsque les nanoparticules sont exposées aux fluides corporels, le revêtement lipophile est métabolisé pour libérer le noyau de taurolidine, et dans laquelle facultativement le noyau de taurolidine s'hydrolyse spontanément pour activer les fractions méthylol lorsque le revêtement lipophile est métabolisé.
